# EUROPEAN PATENT SPECIFICATION

(11) **EP 1 861 504 B1**
(45) Date of publication and mention of the grant of the patent: **16.12.2009**
(21) Application number: 06716639.7
(22) Date of filing: 03.03.2006
(51) Int. Cl.: C12P 21/00

(54) **GLYCOENGINEERING IN MUSHROOMS**
GLYCOENGINEERING IN PILZEN
GLYCOINGÉNIERIE EN CHAMPIGNONS

(30) Priority: 07.03.2005 EP 05075551
(43) Date of publication of application: 05.12.2007
(73) Proprietor: Plant Research International B.V., 6708 PB Wageningen (NL)
(72) Inventor: ROUWENDAL, Gerard, Johan, Adolph, NL-6666 HM Heteren (NL); FLORACK, Dionisius, Elisabeth, Antonius, NL-6708 MD Wageningen (NL); BOSCH, Hendrik, Jan, NL-6708 NA Wageningen (NL); LUGONES, Luis, Gast n, NL-3981 CX Bunnik (NL)
(74) Representative: Hatzmann, Martin
(86) International application number: PCT/NL2006/000112
(87) International publication number: WO 2006/096050

(56) References cited:
- EP-A- 0 570 096
- WO-A2-02/00879
- CHOI ET AL: "Use of combinatorial genetic libraries to humanize N-linked glycosylation in the yeast Pichia pastoris" PROCEEDINGS OF THE NATIONAL ACADEMY OF SCIENCES, USA, vol. 100, 2003, pages 5022-5027, XP002267831 cited in the application

## Description

The invention relates to the field of genetic engineering, more specifically to the field of production of glycoproteins, more specifically to the engineering of the glycosylation pattern of glycoproteins.

Glycoproteins are proteins which have undergone so-called posttranslational modification, in the sense that sugar groups are covalently coupled to the protein. It was found that approximately 50% of the proteins produced by a living cell undergo glycosylation. Further, it appeared that the type of glycosylation differs among the eukaryotes, meaning that different types of sugar moieties are used and/or a different order of attaching the moieties to the protein backbone, thereby forming a variety of branching structures. Glycosylation of proteins is highly regulated and changes during differentiation, development, under different physiological-and cell culture-conditions and in disease. The glycosylation of recombinant proteins, especially those destined for potential administration to human subjects, is of critical importance, while it affects many properties of the (glyco)protein, such as proper folding, protease resistance/sensitivity, intracellular trafficking and compartmentalization, intermolecular affinities and tissue targeting. Glycosylation further profoundly affects biological half-life, activity, function, solubility, clearance from circulation, and crucial for intended pharmaceutical glycoproteins, antigenicity.

The cells of nonhuman species do not glycosylate their proteins in the same way as human cells do. In many cases, the differences are profound. Overall, the species most distant to humans in evolutionary terms, such as bacteria, yeasts, fungi, insects and plants-the species used most commonly in expression systems-have glycosylation repertoires least like our own.

Monoclonal antibodies secreted by recombinant cell expression systems or transgenic organisms nearly always contain a single glycosylation site at Asn 297, a site located in the CH2 domain within the Fc region of the molecule. Monoclonal antibodies lacking the N-linked glycan in the CH2 domain exhibit normal antigen binding mediated by hypervariable regions of the Fab portions of the molecule, but are deficient in Fc effector functions such as antibody dependent cellular cytotoxicity (ADCC) and complement mediated lysis (CDL). These and other effector functions require molecular recognition between the Fc domain of the IgG molecule and proteins such as Fc receptors on the surfaces of immunocytes (ADCC) or soluble proteins that initiate the complement cascade (CML). Glycosylation is important to antibody effector function, such as ADCC and CML, that mediate killing of tumor cells by antibodies developed as oncolytics.

Many expression systems are nowadays employed or explored as biopharmaceutical production platforms (*E. coli*, CHO cells, insect cells, plants, algae, yeasts, filamentous fungi, etc.). Suitability is determined by a lot of parameters, like for example safety, efficacy, costs and not in the least by posttranslational modifications that are either required or that need to be absent.

Yeast expression systems, and *Pichia pastoris* in particular are being explored as potential production platforms for biopharmaceuticals with special requirements with regard to N-glycosylation. As discussed above, the actual composition of this posttranslational modification is essential to certain therapeutic approaches involving monoclonal antibodies, especially those requiring action of the patient's immune system. To this end, fundamental changes had to be established in the N-glycosylation pathway of this yeast.

Yet, there is a need for a system which would provide 'human-like' glycosylation effectively, at low cost and with high yield.

### SUMMARY OF THE INVENTION

The present inventors observed that several basidiomycetes are devoid of hypermannosylated or complex N-glycans and mainly contain high-mannose N-glycans in the range from Man9 down to Man5. It was found that the mere introduction of a heterologous GnT-I activity, without any additional interference with the N-glycosylation pathway, allows for synthesis of the hybrid structure GlcNAc(Man)₅(GlcNAc)₂ (also referred to as GnM5) in basidiomycetes. This hybrid structure can be further modified *in vivo* or *in vitro* to obtain a recombinant glycoprotein with mammalian-type N-glycosylation pattern.

### DESCRIPTION OF THE FIGURES

Fig. 1. MALDI-TOF mass spectra of (M + Na)⁺ adducts of N-glycans from fruiting bodies of *S*. *commune* wild-type (panel A) and transgenic line Sc2 (panel B) transformed with the human GnT-I gene. The arrow indicates the ion corresponding to GlcNAc(Man)₅(GlcNAc)₂ at m/z 1460.5
Fig. 2. A detail of MALDI-TOF mass spectra shown in Figure 1. *S*. *commune* wild-type (panel A) and transgenic line Sc2 transformed with the human GnT-I gene (panel B). The arrow indicates the ion corresponding to GlcNAc(Man)₅(GlcNAc)₂ at m/z 1460.5
Fig. 3. MALDI-TOF mass spectra of (M + Na)⁺ adducts of N-glycans from fruiting bodies of *S*. *commune* wild-type (panel A) and transgenic line Sc12 (panel B) transformed with the human GnT-I gene. The arrow indicates the ion corresponding to GlcNAc(Man)₅(GlcNAc)₂ at m/z 1460.5

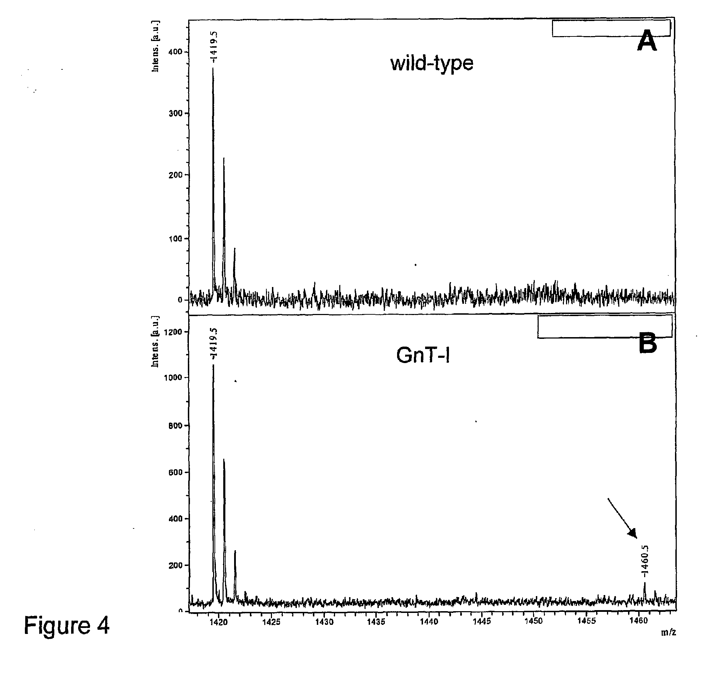

Fig. 4. A detail of MALDI-TOF mass spectra shown in Figure 3. *S*. *commune* wild-type (panel A) and transgenic line Sc2 transformed with the human GnT-I gene (panel B). The arrow indicates the ion corresponding to GlcNAc(Man)₅(GlcNAc)₂ at m/z 1460.5

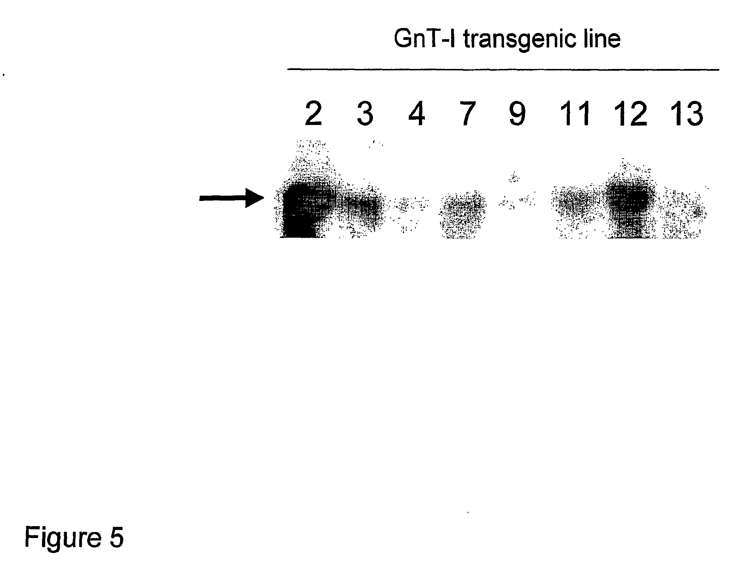

Fig. 5. Northern blot of total RNA purified from mycelia of eight *S. commute* transformants containing the hGnT-I gene using a fragment of this gene as probe. The band indicated by the arrow represents the transcript of GnT-1.
Fig. 6 Nucleotide sequence of a SaII/EcoRI fragment comprising the *Schizophylluni commerce gpd* promoter, the cDNA encoding the human GnT-I and a terminator sequence.

### DETAILED DESCRIPTION OF THE INVENTION

The glycosylation pattern of human proteins differs from the glycosylations that can be seen in proteins expressed in often used eukaryotic protein expression systems, such as yeasts, plants and mammalian cells (for a review see for example Spiro (2002), Glycobiology Vol.12, no. 4, pp.43R-56R).

Bacteria, as being mostly used for large scale recombinant protein production, have the disadvantage that they do not glycosylate the recombinantly expressed proteins.

As expected, glycosylation in mammalian cell expression systems most resembles human glycosylation. However, mammalian cell lines that are able to replicate human-like glycoprotein processing have several drawbacks including low protein titers, long fermentation times, heterogeneous products, and ongoing viral containment issues. For example, it appeared that CHO cells which are genetically engineered to produce large quantities of a specific protein often do not maintain the proper level of glycosylation. This results in low yields of usable product, which contributes to the cost and complexity of producing these glycoproteins.

Glycosylation in plants has been proposed as an alternative, but achieving the desired glycosylation patterns remains a problem with these systems and is a significant barrier to their widespread adoption for manufacturing proteins.

Yeast and filamentous fungi are robust industrial fermentation organisms that can be grown to high cell density in chemically defined medium. However, glycoproteins derived from fungal and yeast expression systems contain non-human N-glycans of the high mannose type, which are immunogenic in human and thus of limited therapeutic value. Yeast and mammals share initial steps of protein N-glycosylation, which involves the site-specific transfer of (GlcNAc)3-(Man)9-(GlcNAc)2 from the luminal side of the endoplasmic reticulum (ER) to the *de novo* synthesized protein. Subsequent trimming by glucosidases I and II and a specific ER-residing α-1,2,-mannosidase leads to the formation of a (Man)₈(GleNAc)₂ structure (isomer ManBB), the N-glycan found on most glycoproteins leaving the ER. After the export of predominantly (Man)₈-(GlcNAc)₂-containing glycoproteins to the Golgi, N-glycan processing pathways diverge notably between mammals and yeast. The human Golgi contains several α-1,2-mannosidases which remove mannose to yield Mans(GlcNAc)2 (also referred to as Man5). The Man5 structure is the preferred acceptor for N-acetylglucosaminyl-transferase I (GnT I), yielding the hybrid structure GlcNAc(Man)₅(GlcNAc)₂ (also referred to as GlcNAcMan5 or GnM5),which is the precursor for complex N-glycans. In contrast, N-glycosylation in yeast (e.g. *Saccharomyces cerevisiae* and *Pichia pastoris*) does not involve removal of mannose residues but the addition of numerous mannose residues. This often leads to hypermannosylated N-gleans with more than 100 mannose residues. Baker's yeast glycosylation patterns are highly immunogenic in mammals (Balou, C.E. (1982) In: Strathern, J.N. et al. (eds.) The Molecular Biology of The Yeast Saccharomyces. Metabolism and Gene Expression. Cold Spring Harbor Laboratory Press, pp. 335-360). Furthermore, the high mannose type N-glycans of yeast do not serve as acceptor for GnT- I and therefore do not allow to serve as precursors for complex N-glycans.

Several attempts have been undertaken to 'humanize' the glycosylation pathway in filamentous fungi and yeast. In the first of these studies it was attempted to use *Aspergillus nidulans* as host for expression of the full-length rabbit GnT-I (Kalsner et al., 1995). GnT-I activity was found intracellularly, but no *in vivo* transfer of GlcNAc residues to fungal N-glycans was detected, possibly due to insufficient levels of ManS(GlcNac)2 acceptor. In 1997, Maras et al. showed that *Trichoderma reesei* contained the acceptor required for GnT-I activity. Later, rat and human GnT-I genes were expressed in the yeast *Saccharomyces cerevisiae* and the filamentous fungus *T. reesei,* respectively, but successful *in vivo* transfer of GlcNAc residues was only detected in *T. reesei* (Yoshida et al., 1999; Maras et al., 1999). Again, the lack of suitable acceptor combined with the absence of a Golgi UDP-GlcNAc transporter will prevent the addition of GlcNAc to yeast N-glycans. Furthermore, only a minor fraction of *T. reesei* oligosaccharides released from *T. reesei* glycoproteins was found to be Man5(GlcNAc)₂. More abundant were the oligosaccharides with 6 to 9 mannose residues and N-glycans with more than 9 mannose residues were also found (see US5,834,251). In general, high mannose structures are rapidly cleared in humans. And, especially the ultrahigh mannose structures (more than 9 mannose residues) are immunogenic.

Glyco-engineering of fungal N-glycans in the yeast *Pichia pastoris* required quite a number of radical changes to the natural N-glycosylation machinery, because the wild-type strains produce N-linked oligosaccharides that can not act as acceptors for the plant and mammalian enzymes constituting the N-glycosylation pathway geared towards the synthesis of complex N-glycans (Choi et al., 2003; Hamilton et al., 2003). First of all, a knockout was made of the Och1p gene that encodes the α-1,6-mannosyltransferase catalyzing the addition of a mannose residue that initiates the addition of yet more mannose sugars to the growing N-glycans structure. Knocking out this gene greatly reduced the synthesis of the highly undesirable hypermannosylated N-glycans, but required the introduction of an ER-localized heterologous α-1,2-mannosidase I to attain yeast strains producing abundant Man5 N-glycan structures that constitute the precursor for GnT-I. Expression of heterologous GnT-I genes in the double transformants yielded a number of strains capable of transferring a GlcNAc amino sugar to Man5. However, the efficiency was low and required the introduction of a fourth transgene encoding the *Kluyveromyces lactis* UDP-GlcNAc transporter to achieve almost complete conversion of the Man5 N-glycan to the hybrid GnM5. Further genetic engineering using heterologous mannosidase-II and GnT-II genes provided strains capable of synthesizing uniform Gn2Man3 structures. The employment of an alg3 mutant strain allowed the development of *P. pastoris* strains that synthesize homogeneous Gn2Man3 N-glycans without using a mannosidase-II activity. The lack of Alg3 activity generates N-glycans that are readily trimmed down to a Man3 structure by a transgene encoded α-1,2-mannosidase-I activity that has been introduced in these strains (Bobrowicz et al., 2004). In fact, these authors showed that expression of a Golgi-localized hybrid enzyme consisting of UDP-glucose 4-epimerase and β-1,4-galactosyltransferase activities could be used to produce bi-antennary, fully galactosylated N-glycans in this yeast.

Thus, whereas fungi and mammals share initial steps of protein N-glycosylation, the abundancy of hypermannosylated N-glycans and the lack of precursors for complex 'mammalian-type" N-glycans make them unattractive for the production of human recombinant glycoproteins.

The present inventors found that several basidiomycetes are devoid of hypermannosylated or complex N-glycans containing high-mannose and only contain N-glycans in the range from Man9 down to Man5. The presence of significant quantities of Man5 N-glycans suggests the presence of one or more α-1,2-mannosidases in the ER and/or Golgi that trim the Man9 structure down to Man5 and intermediate structures. Further, it appeared that basidiomycetes, at least two of them of which the genome has been completely sequenced *(Coprinus cinereus* and *Phanerochaete chrysosporium),* do not contain genes coding for enzymes involved in complex N-glycan synthesis. Unlike the filamentous fungi and yeast expression systems described above, it appeared unnecessary in basidiomycetes to (genetically) interfere with the endogenous N-glycosylation pathway to allow mammalian-type N-glycosylation, more specifically to allow for the formation of the hybrid structure GlcNAcMan₅(GlcNAc)₂ which is the first committed step towards the synthesis of hybrid and complex N-glycans. As is shown herein, the mere introduction of a heterologous GnT-I activity, without any additional interference with the N-glycosylation pathway, allows for synthesis of GIcNAc(Man)₅(GleNAc)₂ (GnM5) in basidiomycetes. Accordingly, the invention relates to a method for producing a glycosylated protein in a recombinant host cell, wherein said host cell is a basidiomycete provided with UDP-N-acetylglucosamine : alpha-3-D-mannoside beta-1,2-N-acetylglucosaminyltransferase (GnT-I; EC 2.4.1.101) activity. GnT-I catalyses the following reaction: UDP-N-acetyl-D-glucosamine + 3-(α-D-mannosyl)-B-D-mannosyl-R → UDP + 3-(2-[N-acetyl-β-D-glucosaminyl]-α-D-mannosyl)-β-D-mannosyl-R. GnT-I is very specific and only transfers GlcNAc to the less periferic α-1,3-linked mannose of a ManS(GlcNAc)2 structure. Other common names for GnT-1 are: α-1,3-mannosyl-glycoprotein 2-β-N-acetylglucosaminyltransferase; N-acetylglucosaminyltransferase I; N-glycosyloligosaccharide-glycoprotein N-acetylglucosaminyltransferase I; uridine diphosphoacetylglucosamine-α-1,3-mannosylglycoprotein β-1,2-N-acetylglucosaminyltransferase; UDP-N-acetylglucosaminyl:α-3-D-mannoside 6-1,2-N-acetylglucosaminyltransferase I and α-1,3-mannosyl-glycoproteinβ-1,2-N-acetylglucosaminyltransferase. Furthermore, the invention provides a method for expressing a heterologous glycosyltransferase enzyme in a basidiomycete, comprising providing said basidiomycete with a nucleic acid construct encoding GnT-I, preferably human GnT-I and allowing expression of said construct in said basidiomycete. Heterologous glycosyltransferase expression can be used to enzymatically synthesize glycoproteins and glycolipids having desired oligosaccharide moieties in a basidiomycete host cell.

Basidiomycetes are phylogenetically much closer to animals than yeasts (T.L. Smith, Proc. Natl. Acad. Sci. USA, 86 7063 (1989)). In one embodiment, a *Pleurotus* spp. or *Auricularia* spp. is used. Many edible mushrooms belong to the order of Agaricales within the subdivision of Basidiomycetes. The family of Agaricaceae is large and includes many familiar mushrooms. For instance, *Agaricus bisporus* is the common cultivated white button mushroom. *Pleurotus ostreatus,* belonging to the family of Pleurotaceae, is a commercially important edible mushroom commonly known as the oyster mushroom. This fungus is industrially produced as human food, and it accounts for nearly a quarter of the world mushroom production. Members of other families within the subclass of holobasidiomycetida (substantial mushrooms) may of course also be used, such as Schizophyllacea.

The use of basidiomycetes as starting point for the production of proteins with a human-like glycosylation pattern enables a faster time to market due to the above given advantages. Also the fact that the production organism may have a GRAYS (Generally Recognised As Safe) status provides for a fast track route, since time-costing and cumbersome acceptance tests can be avoided. Further, the heterologous production in basidiomycetes poses less environmental risks and can be considered to give less containment issues.

In one embodiment of the invention, a basidiomycete is selected from the group consisting of *Agaricus arvensis, Agaricus bisporus, Agaricus blazei, Agrocybe aegerita, Coprinus cinereus, Lentinus edodes, Lepista nuda, Pleurotus ostreatus, Phanerochaete chrysosporium, Schizophyllum commune, Hypsizygus tessulatus, Pholiota nameko, Boletus edulis, Flammulina velutipes, Hericium erinaceus, Volvariella volvacea, Grifola frondosa, Ganoderma lucidum, Tremella fuciformis, Auricularia auricular, Lyophyllum descastes, Naemataloma sublaterium, Stropharia rugoso-annulata* and *Cordyceps sinense.* In a preferred embodiment, the basidiomycete can be cultured on artificial medium. For example, a *Schizophyllum* spp. is used such as *S. commune.* Furthermore, the use of a sporeless basidiomycete in a method of the invention, such as *Pleurotis ostreatus,* is advantageous because it requires less stringent safety measurements. As the basidiomycete host cell needs to be provided with heterologous GnT-I activity, it is of course required that the basidiomycete can be transformed or otherwise provided with a heterologous DNA construct. Methods to transform basidiomycetes are known in the art (see for example Alves et al., Appl Environ Microbiol. 2004;70(11):6379; Godio et al., Curr Genet. 2004 Oct 5; Schuurs et al., Genetics. 1997;147(2):589). EP-A-0 570 096 describes a basidiomycete host-vector system involving the OCTase gene of Coriolus hirsutus.

According to the invention, the endogenous Man5 N-glycans naturally present in basidiomycetes (or at least part thereof) can be converted to GlcNAcMan5 N-glycans by a heterologous GnT-I activity. This step constitutes the first committed step towards the formation of hybrid and complex N-glycans and as such is required for any subsequent glycoengineering step. It requires the use of a heterologous GnT-I gene that is suitable for expression in basidiomycetes. Gene expression in basidiomycetes requires a certain nucleotide composition and is enhanced by the presence of introns (Schuren and Wessels, 1998; Lugones et al., 1999; Ma et al., 2001; Scholtmeijer et al., 2001). Preferably, the gene encoding GnT-I that is used for expression in a basidiomycete has a G/C content of at least 50% and no stretches of about 10 nucleotides or less with more than 90%A or T. Therefore, genes from heterologous sources may require modification to achieve significant expression in a basidiomycete host cell.

For example, expression of a plant-derived cDNA clone encoding GnT-I like the one from *Arabidopsis thaliana* (Bakker et al., 1999) would require extensive modification of the nucleotide composition to achieve production of the enzyme and alteration of N-glycan patterns in transformed basidiomycetes. A variety of methods, usually involving some type of overlap extension PCR starting from long oligonucleotides, exist that can be employed to build a gene with the desired nucleotide composition. However, it can not be excluded that plant cDNA genes may be found that already fulfil all criteria for adequate expression in basidiomycetes. In particular cDNA genes of monocot origin may be useful in that respect.

The human GnT-I cDNA is especially suitable for practicing the present invention, as its natural nucleotide composition appeared to render it suitable for expression without modification. However, many more cDNA clones encoding other vertebrate GnT-I enzymes would be suitable.

To achieve expression of a suitable GnT-I gene in basidiomycetes, its complete open reading frame is typically cloned into an expression vector suitable for transformation of basidiomycetes. The expression vector preferably also comprises nucleic acid sequences that regulate transcription initiation and termination. It is also preferred to incorporate at least one selectable marker gene to allow for selection of transformants. Expression of a glycosyltransferase can be achieved using a basidiomycete promoter, e.g. a constitutive promoter or an inducible promoter. Especially suited are constitutive promoters derived from genes encoding enzymes involved in the glycolytic pathway. An example of a strong constitutive is the glyceraldehyde-3-phosphate dehydrogenase (gpdA) promoter. This promoter is preferred for constitutive expression when recombinant DNA material is expressed in a basidiomycete host. Other examples are the phosphoglycerate kinase (pgk) promoter, the pyruvate kinase (pki) promoter, TPI, the triose phosphate isomerase (tpi) promoter, the APC synthetase subunit g (oliC) promoter and the acetamidase (amdS) promoter of a basidiomycete (WO96/41882).

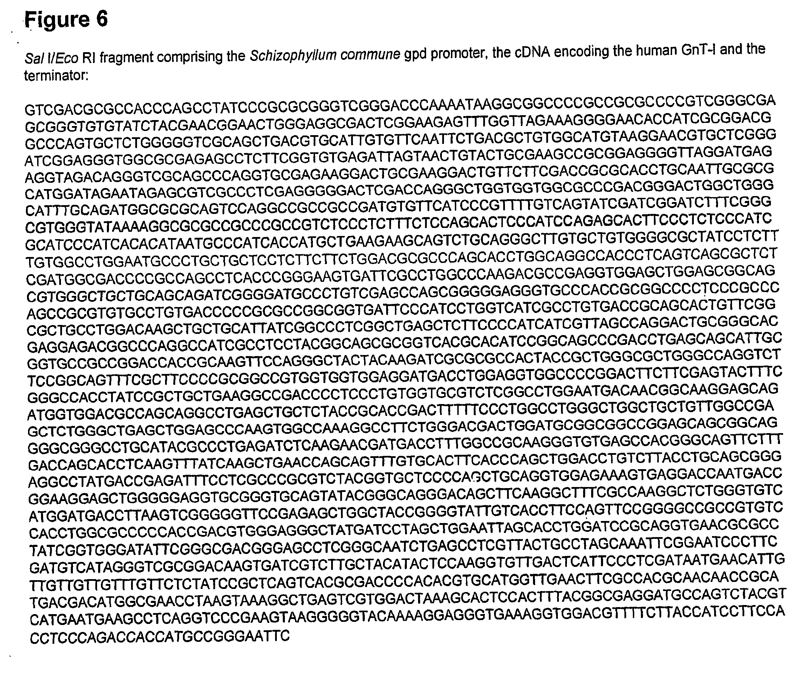

In one embodiment, a basidiomycete *gpd* (glyceraldehyde-3-phosphate dehydrogenase gene) promoter or part thereof is used, for instance (a part of) the *S. commune* gpd promoter or the *Lentinus edodes* gpd promoter. In another embodiment, said gpd promoter includes intron 1 to enhance expression of the heterologous gene (Ma et al., 2001).For example, a basidiomycete is provided with a plasmid comprising the nucleic acid sequence of human GnT-I flanked by a basidiomycete gpd promoter sequence, optionally including intron 1, and a basidiomycete terminator sequence (see for instance Figure 6). In a specific embodiment, GnT-I expression in *S. commune* is achieved by the construction of a vector comprising a *S.commune* derived promoter and a *S. commune* derived terminator with an artificially added intron flanking the insertion site of the GnT-I gene and gene cassette featuring a phleomycin resistance marker (Schuren and Wessels, 1994).

Examples of inducible promoters are the basidiomycete promoters of the following genes: xylanase A (xylA), glucoamylase A (glaA), cellobiohydrolase (cbh), amylase (amy), invertase (suc) and alcohol dehydrogenase alcA, TAKA amylase and amyloglucosidase (ACT) (see WO96/41882).

WO2004/039985 discloses fungal transcription promoters that are suitably used for expression of heterologous genes in a basidiomycete. WO2004/039985 discloses that three genes of the basidiomycete *Agaricus bisporus, abstl, rafe* and *mag2,* are active substantially only during stage 1, or later, of the development of the fruiting body of the fungus. Heterologous DNA under the control of the expression mechanisms of these fungal genes allows for selective expression at this stage of development of the fungus, rather than during growth of the mycelium. In that way, little or no metabolic energy need be diverted from mycelium growth, thereby maximising fruiting body mass and concomitant tissue capable of expressing the heterologous gene once it is switched on.

The construct encoding GnT-I can be transferred to a basidiomycete according to standard procedures. In one embodiment, protoplasts of basidiomycete monokaryon are transformed as described in Schuren and Wessels (1994). Transformants can be selected on selection medium. After some time, mycelial plugs can be harvested and cultured further on fresh medium.

As said, the invention allows for the recombinant production of a protein of interest with a human-like N-glycosylation pattern in a basidiomycete. In one embodiment, a protein of interest is a glycoprotein, such as an antibody (immunoglobulin; Ig) or hormone that is to be provided to a patient in need of the protein, especially for therapeutic reasons.

In one embodiment, a method of the invention provides for a glycosylated Ig molecule or fragment thereof. For example, the antibody is a mammalian IgM, IgA, IgG or IgE, or a fragment thereof. The carbohydrate content of the Ig's ranges form 12-14%. Preferably, the antibody comprises an Fc region. The IgG-Fc region is a homodimer comprised of inter-chain disulphide bonded hinge regions, glycosylated CH2 domains, bearing N-linked oligosaccharide at asparagine 297 and non-covalently paired CH3 domains. Effector mechanisms mediated through FcgRI, FcgRII, FcgRIII and C1q have been shown to be severely compromised for aglycosylated or deglycosylated forms of IgG. Multiple non-covalent interactions between the oligosaccharide and the protein result in a reciprocal influences on conformation. The site of oligosaccharide attachment, Asn 297, is proximal to the N-terminal region of the CH2 domain, from which point it "runs forward" such that terminal sugar residues are exposed at the CH2/CH3 domain interface. In a particularly preferred embodiment, the invention provides for the production of a monoclonal antibody in a basidiomycete host cell.

Examples of glycohormones wherein glycosylation plays a role in the properties of such hormone, especially the therapeutic properties, include erythropoietin (EPO), human choriogonadotropin (HCG), follitropin (FSH), thyrotropin (TSH) and lutropin (LH). Especially, those proteins that require targeting to specific organs or cells through sugar-lectin recognition are useful protein substrates for modification of glycosylation patterns according to the invention. The method of the invention is also useful for the manufacture of a protein for use in enzyme suppletion or replacement therapy. In such therapy, a an enzyme, that is deficient in a cell or organ, is targeted to the cell or organ by providing the enzyme with an appropriate glycosylation pattern.

Expression of the recombinant protein in a basidiomycete can be achieved using a suitable expression vector in a similar manner as described above for the expression of heterologous GnT-I.

The resulting N-glycosylated protein of interest can be harvested from basidiomycetes according to common procedures involving tissue extraction, centrifugation and chromatographic steps depending on the nature of the protein of interest..

The hybrid structure GleNAc(Man)₅(GlcNAc)₂ produced in the basidiomycete by virtue of the heterologous GnT-I activity, can be modified further, either *in vivo* or *in vitro,* into complex N-glycans using one or more glycosylation modification enzymes. The glycosylation modification enzymes that are useful in the methods of the invention include transferases and mannosidases. Section III of US 5,834,251 provides detailed information regarding glycosylation modification enzymes that can be used for practicing the present invention. For example, the hybrid structure GlcNacMan₅GlcNac₂ produced in the basidiomycete by virtue of the heterologous GnT-I activity may be treated *in vivo* or *in vitro* with α-mannosidase II to yield GlcNacMan₃GlcNac₂. This can subsequently serve as a substrate for GlcNAc-transferase II or galactosyltransferse using UDP-GlcNAc or UDP-Gal, respectively to yield a complex type glycosylation pattern. If the Man-I and Man-II have worked properly, no undesirable mannoses will be left at this stage. Nonetheless, if required any remaining α-1,3 and α-1,6-linked mannose residues can be removed *in vivo* or *in vitro* using an "a-specific" mannosidase such as, for example, the commercially available Jack bean mannosidase, thus converting a hybrid N-glycan structure to a complex one.

The further modification can be performed *in vivo* and/or *in vitro.* Of course, for further modification *in vivo* it is required that the basidiomycete host cell is provided with the necessary enzyme activitie(s). This is achieved by the expression of heterologous gene(s) encoding the enzyme(s).

An other aspect of the invention relates to a basidiomycete host cell comprising GnT-I activity, preferably a basidiomycete expressing human GnT-1. Also provided is the use of a basidiomycete as a host cell for the production of a heterologous N-glycosylated (mammalian) protein. Because of the significant endogenous production of (Man)₅(GlcNAc)₂-N-glycan structures, a basidiomycete provides an advantageous starting point for glycoengineering of complex N-glycans. Also encompassed is the *in vitro* conversion of a glycoprotein comprising a (Man)₅(GlcNAc)₂ structure obtained from a basidiomycete into the hybrid structure GlcNAc(Man)₅(GlcNAc)₂ using (recombinant) GnT-I. Details regarding this *in vitro* conversion can be found in US 5,834,251 and references therein. However, it is of course preferred to use a basidiomycete host cell that comprises GnT-I activity, preferably a basidiomycete expressing human GnT-1.

Also provided is a nucleic acid construct encoding a gpd promoter, with or without intron 1, further comprising a GnT-1 gene and, optionally a terminator sequence. The invention furthermore provides a basidiomycete host cell provided with a nucleic acid construct of the invention.

### EXPERIMENTAL SECTION

### Materials and methods

- cDNA prepared from total RNA of Jurkat cells was used for RT-PCR using primers HsGnTI-up
   (GTGACTCTAGAGGTCTCACATGCTGAAGAAGCAGTCTGCAGG) and HsGnTI-dw (GTGACGGATCCAGGTGCTAATTCCAGCTAGGATCATAG). The fragment was purified from agarose gel, digested with *Xba*I and *Bam*HI and cloned into a pUC19-derived plasmid. A Eco31I/BamHI fragment comprising the complete gene was subsequently cloned downstream of a fragment of the *S. commune* GPD promoter into a vector that also contained the phleomycin resistance marker suitable for selection of transformants in *S. commune* (Schuren and Wessels, 1994).

### Strains, growth conditions and media.

The *S. commune* monokaryons 4-39 (*MATA*41*MAT*B41, CBS 341.81) and 4-40 (MATA43MATB43, CBS 341.81) were used. For the isolation of fruiting bodies, transformants from strain 4-40 were crossed with compatible wild type strain 4-39 and grown for one week at 24°C in the light.
*S. commune* was grown in minimal medium (MM) either solidified or not solidified with 1.5% agar (Dons *et al.,* 1979). For transformation, strain 4-40 was grown from a mycelial homogenate for 2 days at 24°C and 225 rpm in 100 ml of MM in 250-ml flasks. For RNA isolation, colonies were grown for 2 to 3 days on the surface of a perforated polycarbonate (PC) membrane (diameter 76 mm, 0.1-µm pores; Poretics) that was positioned on solidified MM.

### Transformation of S. commune

*S. commune* strain 4-40 was transformed as described previously (Schuren and Wessels, 1994), except that it was protoplasted in 1 M MgSO₄ containing 1 mg.mL⁻¹ of lysing enzymes from *Trichoderma harzianum* (Sigma). Then, 5 to 10 µg of DNA were added to 3 x 10⁷ protoplasts in 100 µL of 1 M sorbitol. Transformants were selected on MM plates containing 25 µg.mL⁻¹ of phleomycin (Cayla, Toulouse, France). To reduce the exposure to the mutagenic phleomycin, putative resistant colonies were transferred to MM without the agent. To this end, single hyphae were isolated using an inverted microscope to visualize them. Colonies formed departing from these single hyphae were checked for phleomycin resistance.

### Northern blot analysis

Total RNA was isolated from mycelium that had been ground in liquid nitrogen by using Trizol reagent (Gibco-BRL) following the manufacturers protocol. RNA was separated on a 1% formaldehyde gel and blotted onto Hybond-N⁺ membrane (Amersham) according to Sambrook et al. (1989). The RNA was hybridized with a ³²P-labeled DNA probe at 65°C as described by Church and Gilbert (1984).

### N-glycan purification

Mature and developing fruiting bodies were harvested, quickly frozen in liquid nitrogen and powderized using mortar and pestle. The extraction was done by vigorous vortexing of 100-250 mg of powdered material with 750 µL of buffer 50 mM HEPES-KOH pH 7.5, 20 mM sodium metabisulphite, 5 mM EDTA, 0.1% SDS, 1.7% insoluble polyvinylpolypyrrolidone (PVPP) at room temperature in a 2 mL Eppendorf tube. The debris was removed from the extract by two successive 5 min centrifugations at 20,000 g and 5°C. Total protein was pelleted by 5 min centrifugation following addition of TCA and precipitation on ice for at least 30 min or overnight at 4°C. The pellets were washed twice with 90% acetone and allowed to air-dry. The pellets were resuspended in 0.5 mL of 10 mM HCl and 1.5 mg.mL⁻¹ of pepsin using an Eppendorf micropestle treatment followed by 15-30 min treatment in a sonification bath. Pepsin treatment was continued out for 24 h at 37°C, and then terminated by neutralization to pH 7 with 1 N NH₄OH followed by 10 min heating at 95°C. The resulting (glyco)peptide solution was freeze-dried after removal of insoluble material via centrifugation and the residue dissolved in 0.5 mL sodium phosphate buffer pH 7.5, 1% Nonidet P-40 depending on the enzyme used in the next step. The N-glycans were released from the peptide backbone by 24 h treatment at 37°C with 4.5 mU PNGase F (New England Biolabs). The N-glycans were purified away from the bulk of the peptides by first passing them through a 2 mL Dowex 50 AG 50W-X2 (Bio-Rad) and then passed over a 500 mg C-18 column (Varian). In the final purification step the eluate from the C18 column was applied to an 8-mL 150 mg Ultra-Clean Carbograph column (Alltech) from which the bound N-glycans were eluted in 25% acetonitrile. The oligosaccharides were concentrated by freeze-drying. For galactosidase treatments, N-glycans were incubated with 1.5 mU *Streptococcus pneumoniae* β1,4-galactosidase (Calbiochem) in 50 mM sodium phosphate buffer pH 6.0 and purified away from salts and enzyme using a 150 mg Ultra-Clean Carbograph column and concentrated by freeze-drying. The digestion products were analyzed by MALDI-TOF.

### N-glycan analysis

The purified N-glycans were dissolved in 10 µL of 5 mM NaAc and 1 µL of this solution was combined with an equal volume of 10% 2,5-dihydroxybenzoic acid in 50% acetonitrile. Half of this solution was spotted onto a stainless steel sample plate and dried under a stream of air at room temperature. Positive-ion MALDI-TOF spectra of (M+Na)⁺ adducts were recorded on a Bruker Ultraflex MALDI-TOF fitted with delayed extraction, and a nitrogen laser (337nm). Spectra were generated from the sum of 200-300 laser pulses. A maltodextrin series was used as an external molecular weight standard.

### Results

The cDNA clone encoding the human GnT-I was isolated from cDNA made from Jurkat cells. The complete open reading frame was cloned into an expression vector suitable for transformation of basidiomycetes like *Schizophyllum commune.* This vector comprised a *S*. *commune* derived promoter sequence (sequence see appendix) and a *S*. *commune* derived terminator with an artificially added intron flanking the insertion site of the GnT-I gene and gene cassette featuring the phleomycin resistance marker (Schuren and Wessels, 1994).
The construct was used to transform protoplasts of *S*. *commune* monokaryon 4-40 as described (Schuren and Wessels, 1994) and transformants were selected on MM medium containing 25 µg.mL⁻¹ phleomycin. After one week mycelial plugs were taken and transferred to fresh MM medium without phleomycin. Mycelium was harvested and Northern blot analysis of total RNA isolated from 8 transformants revealed variable expression of the GnT-I transgene and the highest expression was found in transformants 2 and 12. The transformants was crossed with the compatible monokaryon 4-39 and grown until harvesting of the developing and mature fruiting bodies. The harvested dikaryotic material was extracted to purify total protein and then N-glycans were released from the protein backbone by PNGase F and purified before MALDI-TOF analysis.

MALDI-TOF analysis of the wild-type and transformants 2, 3, 4, 12 and 13 revealed a number of very abundant N-glycans in all samples representing high-mannose oligosaccharides (Man)₉(GlcNAc)₂ down to (Man)₅(GlcNAc)₂ (Table 1).

**Table 1. m/z values of (M + Na)+ ions of typical N-glycans encountered in wild-type S. commune.**

| N-glycans | m/z |
|---|---|
| (Man)₅(GlcNAc)₂ | 1257.4 |
| (Man)₆(GlcNAc)₂ | 1419.5 |
| (Man)₇GlcNAc)₂ | 1581.5 |
| (Man)₈(GlcNAc)₂ | 1743.6 |
| (Man)₈GlcNAc)₂ | 1905.6 |

**Table 2. m/z values of (M + Na)+ ions of typical N-glycans encountered in transgenic S. commune.**

| N-gycans | m/z |
|---|---|
| (Man)₂(GlcNAc)₂ | 1257.4 |
| (Man)₆(GlcNAc)₂ | 1419.5 |
| GlcNAc(Man)₅(GlcNAc)₂ | 1460.4 |
| (Man)₇GlcNAc)₂ | 1581.5 |
| (Man)₈(GlcNAc)₂ | 1743.6 |
| (Man)₉GlcNAc)₂ | 1905.6 |

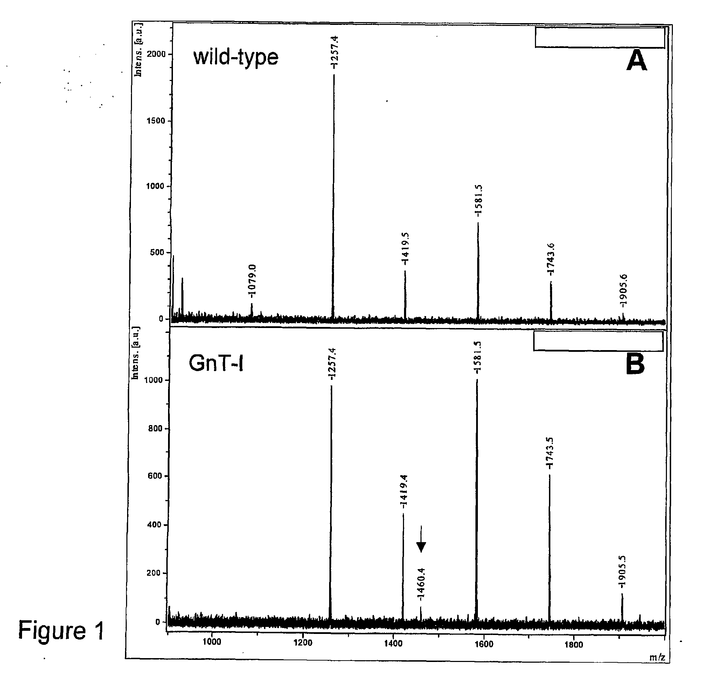

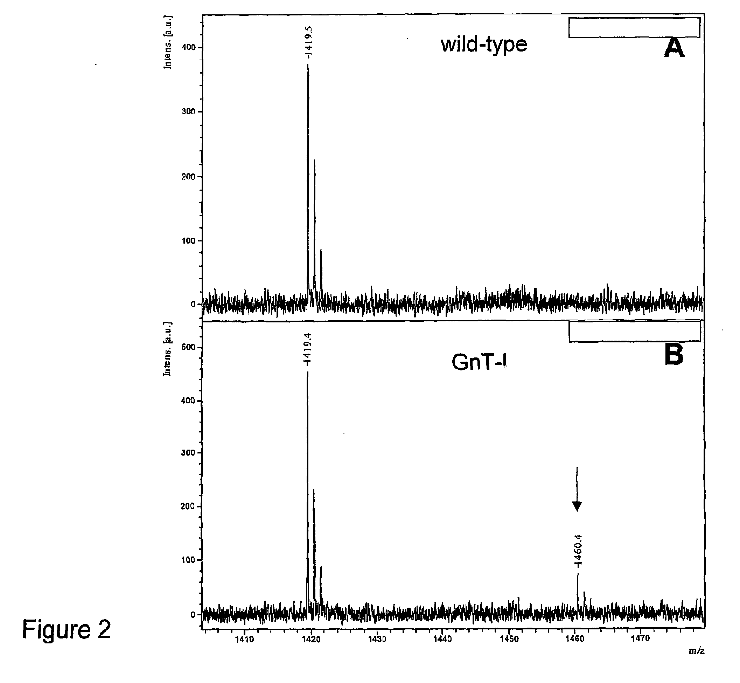

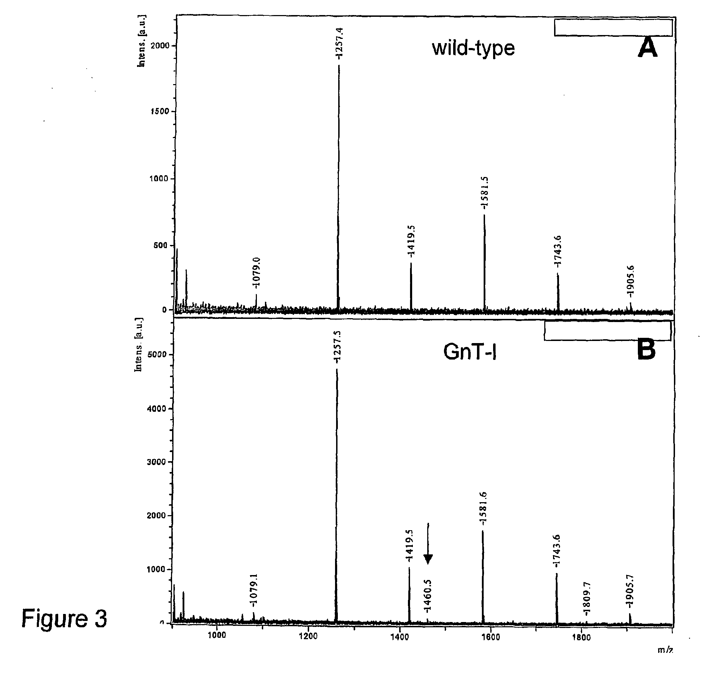

Two transformants - 2 and 12 - displayed the presence of a small, but significant amount of a product with a m/z of 1460.5 that is GlcNAc(Man)₅(GlcNAc)₂ (Figs. 1 and 2). These data demonstrate that GnT-I is expressed and active in *S*. *commune* and that a detectable fraction of the endogenous GnT-I acceptor (Man)₅(GlcNAc)₂ (Man5) has been converted to GlcNAc(Man)₅(GlcNAc)₂ (GnM5). Since only the two transformants with the highest level of GnT-I mRNA contained the novel hybrid N-glycan (Fig. 3), it is conceivable that further increasing the level of expression will lead to increased GnM5 levels.

### References

Bakker H, Lommen A, Jordi W, Stiekema W, Bosch D (1999) An Arabidopsis thaliana cDNA complements the N-acetylglucosaminyltransferase I deficiency of CHO Lec1 cells. Biochem Biophys Res Commun 261: 829-32
Bobrowicz P, Davidson RC, Li H, Potgieter TI, Nett JH, Hamilton SR, Stadheim TA, Miele RG, Bobrowicz B, Mitchell T, Rausch S, Renfer E, Wildt S (2004) Engineering of an artificial glycosylation pathway blocked in core oligosaccharide assembly in the yeast Pichia pastoris: production of complex humanized glycoproteins with terminal galactose. Glycobiology 14: 757-66
Choi BK, Bobrowicz P, Davidson RC, Hamilton SR, Kung DH, Li H, Miele RG, Nett JH, Wildt S, Gerngross TU (2003) Use of combinatorial genetic libraries to humanize N-linked glycosylation in the yeast Pichia pastoris. Proc Natl Acad Sci U S A. 2003 100: 5022-7.
Church GM, Gilbert W (1984) Genomic sequencing. Proc Natl Acad Sci USA 81:1991-5.
Dons JJM, De Vries OMH, Wessels JGH (1979) Characterisation of the genome of the basidiomycete Schizophyllum commune. Biochem Biophys Acta 563:100-112.
Hamilton SR, Bobrowicz P, Bobrowicz B, Davidson RC, Li H, Mitchell T, Nett JH, Rausch S, Stadheim TA, Wischnewski H, Wildt S, Gerngross TU (2003) Production of complex human glycoproteins in yeast. Science 301: 1244-6
Kalsner I, Hintz W, Reid LS, Schachter H (1995) Insertion into Aspergillus nidulans of functional UDP-GlcNAc: alpha 3-D- mannoside beta-1,2-N-acetylglucosaminyl-transferase I, the enzyme catalysing the first committed step from oligomannose to hybrid and complex N-glycans. Glycoconj J 12: 360-70
Lugones LG, Scholtmeijer K, Klootwijk R, Wessels JG (1999) Introns are necessary for mRNA accumulation in Schizophyllum commune. Mol Microbiol 32: 681-9
Ma B, Mayfield MB, Gold MH (2001) The green fluorescent protein gene functions as a reporter of gene expression in Phanerochaete chrysosporium. Appl Environ Microbiol 67: 948-55
Maras M, De Bruyn A, Schraml J, Herdewijn P, Claeyssens M, Fiers W, Contreras R (1997) Structural characterization of N-linked oligosaccharides from cellobiohydrolase I secreted by the filamentous fungus Trichoderma reesei RUTC 30. Eur J Biochem 245: 617-25.
Maras M, De Bruyn A, Vervecken W, Uusitalo J, Penttila M, Busson R, Herdewijn P, Contreras R (1999) In vivo synthesis of complex N-glycans by expression of human N-acetylglucosaminyltransferase I in the filamentous fungus Trichoderma reesei. FEBS Lett 452: 365-70
Sambrook J, Fritsch EF, Maniatis T (1989) Molecular cloning: a laboratory manual, 2nd ed. Cold Spring Harbor Laboratory, Cold Spring Harbor. N.Y.
Scholtmeijer K, Wosten HA, Springer J, Wessels JG (2001) Effect of introns and AT-rich sequences on expression of the bacterial hygromycin B resistance gene in the basidiomycete Schizophyllum commune. Appl Environ Microbiol 67: 481-3
Schuren FH, Wessels JG (1994) Highly-efficient transformation of the homobasidiomycete Schizophyllum commune to phleomycin resistance. Curr Genet 26:179-83
Schuren FH, Wessels JG (1998) Expression of heterologous genes in Schizophyllum commune is often hampered by the formation of truncated transcripts. Curr Genet 33: 151-6
Vervecken W, Kaigorodov V, Callewaert N, Geysens S, De Vusser K, Contreras R (2004) In vivo synthesis of mammalian-like, hybrid-type N-glycans in Pichia pastoris. Appl Environ Microbiol 70: 2639-46
Wilson IB, Zeleny R, Kolarich D, Staudacher E, Stroop CJ, Kamerling JP, Altmann F (2001) Analysis of Asn-linked glycans from vegetable foodstuffs: widespread occurrence of Lewis a, core alphal,3-linked fucose and xylose substitutions. Glycobiologyl: 261-74
Yoshida S, Suzuki M, Yamano S, Takeuchi M, Ikenaga H, Kioka N, Sakai H, Komano T (1999) Expression and characterization of rat UDP-N-acetylglucosamine: alpha-3-D-mannoside beta-1,2-N-acetylglucosaminyltransferase I in Saccharomyces cerevisiae. Glycobiology 9: 53-8

## Claims

1. Method for producing a glycoprotein in a recombinant host cell, comprising allowing expression of said glycoprotein in a basidiomycete host provided with N-acetylglucosamine : alpha-3-D-mannoside beta-1,2-N-acetylglucosaminyltransferase (GnT-I; EC 2.4.1.101) activity.

2. Method according to claims 1, wherein said basidiomycete is selected from the group consisting of *Agaricus arvensis, Agaricus bisporus, Agaricus blazei, Agrocybe aegerita, Coprinus cinereus, Lentinus edodes, Lepista nuda, Pleurotus ostreatus, Phanerochaete chrysosporium, Schizophyllum commune, Hypsizygus tessulatus, Pholiota nameko, Boletus edulis, Flammulina velutipes, Hericium erinaceus, Volvariella volvacea, Grifola frondosa, Ganoderma lucidum, Tremella fuciformis, Auricularia auricular, Lyophyllum descastes, Naemataloma sublaterium, Stropharia rugoso-annulata* and *Cordyceps sinense.*

3. Method according to claim 2, wherein said basidiomycete is sporeless.

4. Method according to any of the previous claims, wherein said basidiomycete is provided with a mammalian gene encoding GnT-I activity.

5. Method according to claim 4, wherein the mammalian gene is a human gene.

6. Method according to any one of the previous claims, wherein said glycoprotein is a heterologous protein.

7. Method according to claim 6, wherein said glycoprotein is a hormone, an antibody, a cytokine or an enzyme.

8. Method according to any one of the previous claims, additionally comprising the further modification of said glycoprotein with at least one transferase and/or a mannosidase.

9. Method according to claim 8, wherein said at least one transferase and/or mannosidase is selected from the group consisting of GlcNAc-transferase II, III, IV, V, VI, α-mannosidase II, α-mannosidase III, galactosyltransferase, sialyltransferase

10. Method according to any of the previous claims, wherein at least one of the further modification steps is performed intracellularly in said basidiomycete.

11. Method according to claim 9 or 10, wherein at least one of the further modification steps is performed in vitro.

12. Method for expressing a heterologous GnT-1 enzyme in a basidiomycete, comprising providing said basidiomycete with a nucleic acid construct encoding said enzyme and allowing expression of said construct in said basidiomycete.

13. Nucleic acid construct comprising a promoter sequence allowing for expression in a basiodiomycete, preferably a gpd promoter, further comprising a nucleic acid sequence encoding GnT-1 activity, and optionally further comprising a transcription terminator sequence.

14. Nucleic acid according to claim 13, wherein the nucleic acid sequence encoding GnT-1 activity is the human GnT-1 gene.

15. A recombinant basidiomycete host cell comprising GnT-I activity.

16. A recombinant basidiomycete host cell according to claim 15 expressing human GnT-1.

17. Basidiomycete host cell comprising a nucleic acid construct according to claim 13 or 14.

18. Use of a basidiomycete comprising GnT-I activity as a host for the production of a heterologous N-glycosylated mammalian protein.

19. Use according to claim 18, wherein said basidiomycete expresses human GnT-1.

20. Use according to claim 18 or 19, wherein said basidiomycete comprises a basidiomycete host cell according to claim 15 or 16.

## Patentansprüche

1. Verfahren zur Herstellung eines Glycoproteins in einer rekombinanten Wirtszelle, umfassend das Exprimierenlassen des Glycoproteins in einem Basidiomyceten-Wirt, der mit der Aktivität einer N-Acetylglucasamin:α-3-D-mannosid-β-1,2-N-acetylglucosaminyltransferase (GnT-I; EC 2.4.1. 101) versehen ist.

2. Verfahren gemäß Anspruch 1, wobei der Basidiomycet aus der Gruppe ausgewählt ist, die aus *Agaricus arvensis, Agaricus bisporus, Agaricus blazei, Agrocybe aegerita, Coprinus cinereus, Lentinus edodes, Lepista nuda, Pleurotus ostreatus, Phanerochaete chrysosporium, Schizophyllum commune, Hypsizygus tessulatus, Pholiota nameko, Boletus edulis, Flammulina velutipes, Hericium erinaceus, Volvariella volvacea, Grifola frondosa, Genoderma lucidum, Tremella fuciformis, Auricularia auricula, Lyophyllum decastes, Naematoloma sublaterium, Stropharia rugoso-annulata* und *Cordyceps sinense* besteht.

3. Verfahren gemäß Anspruch 2, wobei der Basidiomycet sporenlos ist.

4. Verfahren gemäß einem der vorstehenden Ansprüche, wobei der Basidiomycet mit einem Säuger-Gen, das GnT-1-Aktivität codiert, versehen ist.

5. Verfahren gemäß Anspruch 4, wobei das Säuger-Gen ein humanes Gen ist.

6. Verfahren gemäß einem der vorstehenden Ansprüche, wobei das Glycoprotein ein heterologes Protein ist,

7. Verfahren gemäß Anspruch 6, wobei das Glycoprotein ein Hormon, ein Antikörper, ein Cytokin oder ein Enzym ist.

8. Verfahren gemäß einem der vorstehenden Ansprüche, das zusätzlich die weitere Modifikation des Glycoproteins mit wenigstens einer Transferase und/oder Mannosidase umfasst.

9. Verfahren gemäß Anspruch 8, wobei die wenigstens eine Transferase und/oder Mannosidase aus der Gruppe ausgewählt ist, die aus GlcNAc-Transferase II, III, IV, V, VI, α-Mannosidase II, α-Mannosidase III, Galactosyltransferase, Sialyltransferase besteht.

10. Verfahren gemäß einem der vorstehenden Ansprüche, wobei wenigstens einer der weiteren Modifikationsschritte intrazellulär in dem Basidiomyceten durchgeführt wird,

11. Verfahren gemäß Anspruch 9 oder 10, wobei wenigstens einer der weiteren Modifikationsschritte in vitro durchgeführt wird.

12. Verfahren zum Exprimierten eines heterologen GnT-1-Enzyms in einem Basidiomyceten, umfassend das Versehen des Basidiomyceten mit einem Nucleinsäurekonstrukt, das das Enzym codiert, und das Exprimlerenlassen des Konstrukts in dem Basidiomyceten.

13. Nucleinsäurekonstrukt, das eine Promotorsequenz, die die Expression in einem Basidiomyceten ermöglicht, vorzugsweise einen gpd-Promotor, umfasst, weiterhin eine Nucleinsäuresequenz, die GnT-1-Aktivität codiert, umfasst und gegebenenfalls weiterhin eine Transcriptionsterminationssequenz umfasst.

14. Nucleinsäure gemäß Anspruch 13, wobei es sich bei der Nucteinsäuresequenz, die die GnT-1-Aktivität codiert, um das humane GnT-1-Gen handelt.

15. Rekombinante Basidiomyceten-Wirtszelle, die GnT-1-Aktivität umfasst.

16. Rekombinante Basidiomyceten-Wirtszelle gemäß Anspruch 15, die humanes GnT-1 exprimiert.

17. Basidlomyceten-Wirtszelle, die ein Nucleinsäurekonstrukt gemäß Anspruch 13 oder 14 umfasst.

18. Verwendung eines Basidiomyceten, der GnT-1-Aktivität umfasst, als Wirt für die Produktion eines heterologen N-glycosylierten Säugerproteins.

19. Verwendung gemäß Anspruch 18, wobei der Basidiomycet humanes GnT-1 exprimiert.

20. Verwendung gemäß Anspruch 18 oder 19, wobei der Basidiomycet eine Basidlomyceten-Wirtszelle gemäß Anspruch 15 oder 16 umfasst.

## Revendications

1. Procédé de production d'une glycoprotéine dans une cellule hôte recombinante, comprenant l'expression de ladite glycoprotéine dans un hôte basidiomycète qui possède une activité de N-acétylglucosamine : alpha-3-D-mannoside bêta-1,2-N-acétylglucosaminyltransférase (GnT-1 ; EC 2.4.1.101).

2. Procédé selon la revendication 1, dans lequel ledit basidiomycète est choisi dans le groupe constitué par *Agaricus arvensis, Agaricus bisporus, Agaricus blazei, Agrocybe aegerita, Coprinus cinereus, Lentinus edodes, Lepista nuda, Pleurotus ostreatus, Phanerochaete chrysosporium, Schizophyllum commune, Hypsizygus tessulatus, Pholiota nameko, Boletus edulis, Flammulina velutipes, Hericium erinaceus, Volvariella volvacea, Grifola frondosa, Ganoderma lucidum, Tremella fuciformis, Auricularia auricula-judae, Lyophyllum decastes, Naematoloma sublaterium, Stropharia rugoso-annulata* et *Cordyceps sinense.*

3. Procédé selon la revendication 2, dans lequel ledit basidiomycète est asporulé.

4. Procédé selon l'une quelconque des revendications précédentes, dans lequel ledit basidiomycète contient un gène de mammifère codant pour l'activité de GnT-1.

5. Procédé selon la revendication 4, dans lequel le gène de mammifère est un gène humain.

6. Procédé selon l'une quelconque des revendications précédentes, dans lequel ladite glycoprotéine est une protéine hétérologue.

7. Procédé selon la revendication 6, dans lequel ladite glycoprotéine est une hormone, un anticorps, une cytokine ou une enzyme.

8. Procédé selon l'une quelconque des revendications précédentes, comprenant en plus la modification supplémentaire de ladite glycoprotéine avec au moins une transférase et/ou une mannosidase.

9. Procédé selon la revendication 8, dans lequel ladite au moins une transférase et/ou mannosidase est choisie dans le groupe constitué par la GlcNAC transférase II, III, IV, V, VI, l'α-mannosidase II, l'α-mannosidase III, la galactosyltransférase, la sialyltransférase.

10. Procédé selon l'une quelconque des revendications précédentes, dans lequel l'une au moins des étapes de modification supplémentaire s'effectue dans le milieu intracellulaire dans ledit basidiomycète.

11. Procédé selon la revendication 9 ou la revendication 10, dans lequel l'une au moins des étapes de modification supplémentaire s'effectue *in vitro.*

12. Procédé d'expression d'une enzyme GnT-1 hétérologue dans un basidiomycète, comprenant l'introduction dans ledit basidiomycète d'un produit d'assemblage d'acides nucléiques codant pour ladite enzyme et permettant l'expression dudit produit d'assemblage dans ledit basidiomycète.

13. Produit d'assemblage d'acides nucléiques, comprenant une séquence promoteur qui permet l'expression dans un basidiomycète, de préférence un promoteur gpd, comprenant en plus une séquence d'acides nucléiques codant pour l'activité de GnT-1, et comprenant en plus facultativement une séquence de terminaison de la transcription.

14. Produit d'assemblage d'acides nucléiques selon la revendication 13, dans lequel la séquence d'acides nucléiques codant pour l'activité de GnT-1 est le gène GnT-1 humain.

15. Cellule hôte recombinante de basidiomycète, comprenant une activité de GnT-1.

16. Cellule hôte recombinante de basidiomycète selon la revendication 15, exprimant la GnT-1 humaine.

17. Cellule hôte de basidiomycète, comprenant un produit d'assemblage d'acides nucléiques selon la revendication 13 ou la revendication 14.

18. Utilisation d'un basidiomycète comprenant une activité de GnT-1 comme hôte pour la production d'une protéine de mammifère hétérologue N-glycosylée.

19. Utilisation selon la revendication 18, dans laquelle ledit basidiomycète exprime la GnT-1 humaine.

20. Utilisation selon la revendication 18 ou la revendication 19, dans lequel ledit basidiomycète comprend une cellule hôte de basidiomycète selon la revendication 15 ou la revendication 16.
